(19) Europäisches Patentamt
European Patent Office
Office européen des brevets

(11) **EP 1 885 439 B1**

(12) **EUROPEAN PATENT SPECIFICATION**

(45) Date of publication and mention
of the grant of the patent:
**08.12.2010 Bulletin 2010/49**

(51) Int Cl.:
***A61N 1/362*** (2006.01)

(21) Application number: **06739182.1**

(22) Date of filing: **22.03.2006**

(86) International application number:
**PCT/US2006/010287**

(87) International publication number:
**WO 2006/115638 (02.11.2006 Gazette 2006/44)**

(54) **APPARATUS FOR TREATMENT OF PROLONGED INTER-ATRIAL DELAY**

VORRICHTUNG ZUR BEHANDLUNG VON LANGEN INTERATRIALEN VERZÖGERUNGEN

APPAREIL DE TRAITEMENT D'UN RETARD INTER-AURICULAIRE PROLONGE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **25.04.2005 US 113809**

(43) Date of publication of application:
**13.02.2008 Bulletin 2008/07**

(73) Proprietor: **CARDIAC PACEMAKERS, INC.
St. Paul, Minnesota 55112-5798 (US)**

(72) Inventors:
• **YU, Yinghong
Shoreview, Minnesota 55126 (US)**
• **DING, Jiang
Shoreview, Minnesota 55109 (US)**
• **MORRIS, Milton M.
Minneapolis, MN 55411 (US)**

(74) Representative: **Pfenning, Meinig & Partner GbR
Patent- und Rechtsanwälte
Joachimstaler Strasse 12
10719 Berlin (DE)**

(56) References cited:
**US-A- 5 902 324          US-A1- 2003 083 700
US-A1- 2004 193 223**

**Description**

FIELD OF THE INVENTION

This invention pertains to methods and apparatus for treating cardiac disease with electrical therapy.

Background

Cardiac rhythm management devices are implantable devices that provide electrical stimulation to selected chambers of the heart in order to treat disorders of cardiac rhythm. A pacemaker, for example, is a cardiac rhythm management device that paces the heart with timed pacing pulses. Pacemakers of this kind are disclosed, for example, in documents US 2003/083700 A1, US 2004/193223 A1 or US-A-5902324. The most common condition for which pacemakers are used is in the treatment of bradycardia, where the ventricular rate is too slow. Atrio-ventricular conduction defects (i.e., AV block) that are permanent or intermittent and sick sinus syndrome represent the most common causes of bradycardia for which permanent pacing may be indicated. If functioning properly, the pacemaker makes up for the heart's inability to pace itself at an appropriate rhythm in order to meet metabolic demand by enforcing a minimum heart rate and/or artificially restoring AV conduction.

Pacing therapy can also be used in the treatment of heart failure, which refers to a clinical syndrome in which an abnormality of cardiac function causes a below normal cardiac output that can fall below a level adequate to meet the metabolic demand of peripheral tissues. When uncompensated, it usually presents as congestive heart failure due to the accompanying venous and pulmonary congestion. Heart failure can be due to a variety of etiologies with ischemic heart disease being the most common. It has been shown that some heart failure patients suffer from intraventricular and/or interventricular conduction defects (e.g., bundle branch blocks) such that their cardiac outputs can be increased by improving the synchronization of ventricular contractions with electrical stimulation. In order to treat these problems, implantable cardiac devices have been developed that provide appropriately timed electrical stimulation to one or more heart chambers in an attempt to improve the coordination of atrial and/or ventricular contractions, termed cardiac resynchronization therapy (CRT). Currently, a most common form of CRT applies stimulation pulses to both ventricles, either simultaneously or separated by a specified biventricular offset interval, and after a specified atrio-ventricular delay interval with respect to the detection of an intrinsic atrial contraction and/or an atrial pace.

Certain patients, in addition to having ventricular conduction problems, have prolonged inter-atrial conduction delays. A prolonged inter-atrial delay compromises the synchronization of atrial and ventricular contractions which can complicate the optimal delivery of CRT. It is this problem with which the present disclosure is primarily concerned.

SUMMARY

Patients with prolonged inter-atrial conduction delays exhibit delayed conduction of excitation from the right atrium to the left atrium. The delay may exist during intrinsic beats in which the excitation originates at the sino-atrial node in the right atrium, during an atrial paced beat in which the excitation originates at a right atrial pacing site, or both. The delayed contraction of the left atrium results in sub-optimal diastolic filling of the left ventricle during atrial systole and, hence, decreased cardiac output. Application of ventricular CRT in these patients in a conventional manner, where the left ventricle is pre-excited with pacing pulses and thus made to contract sooner during a cardiac cycle, may worsen the asynchrony between the left atrium and the left ventricle and even cause the left ventricle to contract before the left atrium. Besides interfering with optimal delivery of CRT, such a reversed AV contraction sequence may have other adverse consequences. The present invention, as defined in claim 1, overcomes these drawbacks.
One way by which an inter-atrial delay may be reduced is to resynchronize the atria by stimulating (i.e., pacing) the left atrium either instead of or in addition to stimulating the right atrium. Identifying the degree of inter-atrial delay which would have a negative impact on the effectiveness of CRT, however, is problematic. Another difficulty is assessing the inter-atrial delay in order to appropriately specify pacing parameters for delivering CRT with or without atrial resynchronization. Described below are methods and apparatus for identifying and assessing an inter-atrial delay by sensing electrical activity in the left atrium and left ventricle and measuring the delay between left atrial and left ventricular contractions, referred to as the LA-LV interval.

BRIEF DESCRIPTION OF THE DRAWINGS

Fig. 1 is a system diagram of exemplary hardware components for delivering cardiac resynchronization therapy.
Fig. 2 illustrates an exemplary algorithm for identifying and treating patients with prolonged inter-atrial conduction delays.

DETAILED DESCRIPTION

Described herein are a method and system for setting the pacing parameters and/or pacing configuration of a cardiac rhythm management device for delivering resynchronization pacing to the left ventricle (LV) and/or the right ventricle (RV) in order to compensate for ventricular conduction delays and improve the coordination of ventricular contractions. Another aspect of the disclosure involves the identification and assessment of inter-atrial conduction delays which can compromise the delivery of CRT by measuring the LA-LV interval. If the LA-LV interval is found to be less than a specified threshold, the patient may be treated with left atrial pacing to restore LA-LV synchrony. Alternatively, some patients may be treated with CRT using a conservative (i.e., long) AV delay interval.

1. Exemplary hardware platform

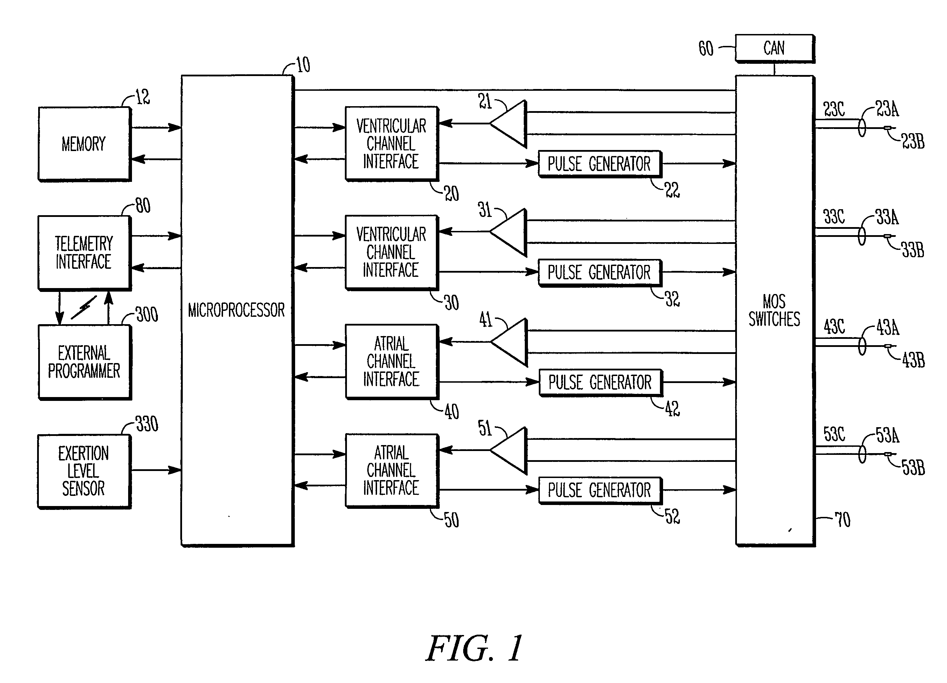

The following is a description of exemplary hardware components used for practicing the present invention. A block diagram of an implantable cardiac rhythm management device or pulse generator having multiple sensing and pacing channels is shown in Fig. 1. Pacing of the heart with an implanted device involves excitatory electrical stimulation of the heart by the delivery of pacing pulses to an electrode in electrical contact with the myocardium. The device is usually implanted subcutaneously on the patient's chest, and is connected to electrodes by leads threaded through the vessels of the upper venous system into the heart. An electrode can be incorporated into a sensing channel that generates an electrogram signal representing cardiac electrical activity at the electrode site and/or incorporated into a pacing channel for delivering pacing pulses to the site.

The controller of the device in Fig. 1 is made up of a microprocessor 10 communicating with a memory 12 via a bidirectional data bus, where the memory 12 typically comprises a ROM (read-only memory) and/or a RAM (random-access memory). The controller could be implemented by other types of logic circuitry (e.g., discrete components or programmable logic arrays) using a state machine type of design, but a microprocessor-based system is preferable. As used herein, the programming of a controller should be taken to refer to either discrete logic circuitry configured to perform particular functions or to the code executed by a microprocessor. The controller is capable of operating the pacemaker in a number of programmed modes where a programmed mode defines how pacing pulses are output in response to sensed events and expiration of time intervals. A telemetry interface 80 is provided for communicating with an external programmer 300. The external programmer is a computerized device with an associated display and input means that can interrogate the pacemaker and receive stored data as well as directly adjust the operating parameters of the pacemaker. As described below, in certain embodiments of a system for setting pacing parameters, the external programmer may be utilized for computing optimal pacing parameters from data received from the implantable device over the telemetry link which can then be set automatically or presented to a clinician in the form of recommendations.

The embodiment shown in Fig. 1 has four sensing/pacing channels, where a pacing channel is made up of a pulse generator connected to an electrode while a sensing channel is made up of the sense amplifier connected to an electrode. A MOS switching network 70 controlled by the microprocessor is used to switch the electrodes from the input of a sense amplifier to the output of a pulse generator. The switching network 70 also allows the sensing and pacing channels to be configured by the controller with different combinations of the available electrodes. The channels may be configured as either atrial or ventricular channels allowing the device to deliver conventional ventricular single-site pacing with or without atrial tracking, biventricular pacing, or multi-site pacing of a single chamber. In an example configuration, a left atrial (LA) sensing/pacing channel includes ring electrode 53a and tip electrode 53b of bipolar lead 53c, sense amplifier 51, pulse generator 52, and a channel interface 50, and a right atrial (RA) sensing/pacing channel includes ring electrode 43a and tip electrode 43b of bipolar lead 43c, sense amplifier 41, pulse generator 42, and a channel interface 40. A right ventricular (RV) sensing/pacing channel includes ring electrode 23a and tip electrode 23b of bipolar lead 23c, sense amplifier 21, pulse generator 22, and a channel interface 20, and a left ventricular (LV) sensing/pacing channel includes ring electrode 33a and tip electrode 33b of bipolar lead 33c, sense amplifier 31, pulse generator 32, and a channel interface 30. The channel interfaces communicate bi-directionally with a port of microprocessor 10 and include analog-to-digital converters for digitizing sensing signal inputs from the sensing amplifiers, registers that can be written to for adjusting the gain and threshold values of the sensing amplifiers, and registers for controlling the output of pacing pulses and/or changing the pacing pulse amplitude. In this embodiment, the device is equipped with bipolar leads that include two electrodes which are used for outputting a pacing pulse and/or sensing intrinsic activity. Other embodiments may employ unipolar leads with single electrodes for sensing and pacing. The switching network 70 may configure a channel for unipolar sensing or pacing by referencing an electrode of a unipolar or bipolar lead with the device housing or can 60.

The controller controls the overall operation of the device in accordance with programmed instructions stored in memory. The controller interprets electrogram signals from the sensing channels and controls the delivery of paces in accordance with a pacing mode. An exertion level sensor 330 (e.g., an accelerometer, a minute ventilation sensor, or other sensor that measures a parameter related to metabolic demand) enables the controller to adapt the atrial and/or ventricular pacing rate in accordance with changes in the patient's physical activity, termed a rate-adaptive pacing mode. The sensing circuitry of the device generates atrial and ventricular electrogram signals from the voltages sensed by the electrodes of a particular channel. An electrogram is analogous to a surface EKG and indicates the time course and amplitude of cardiac depolarization and repolarization that occurs during either an intrinsic or paced beat. When an electrogram signal in an atrial or ventricular sensing channel exceeds a specified threshold, the controller detects an atrial or ventricular sense, respectively, which pacing algorithms may employ to trigger or inhibit pacing.

In one embodiment, the exertion level sensor is a minute ventilation sensor which includes an exciter and an impedance measuring circuit. The exciter supplies excitation current of a specified amplitude (e.g., as a pulse waveform with constant amplitude) to excitation electrodes that are disposed in the thorax. Voltage sense electrodes are disposed in a selected region of the thorax so that the potential difference between the electrodes while excitation current is supplied is representative of the transthoracic impedance between the voltage sense electrodes. The conductive housing or can may be used as one of the voltage sense electrodes. The impedance measuring circuitry processes the voltage sense signal from the voltage sense electrodes to derive the impedance signal. Further processing of the impedance signal allows the derivation of signal representing respiratory activity and/or cardiac blood volume, depending upon the location the voltage sense electrodes in the thorax or cardiac anatomy. (See, e.g., U.S. Patent Nos. 5,190,035 and 6,161,042, assigned to the assignee of the present invention and hereby incorporated by reference.) If the impedance signal is filtered to remove the respiratory component, the result is a signal that is representative of blood volume in the heart at any point in time, thus allowing the computation of stroke volume and, when combined with heart rate, computation of cardiac output.

2. Cardiac resynchronization pacing therapy

Cardiac resynchronization therapy is most conveniently delivered in conjunction with a bradycardia pacing mode. Bradycardia pacing modes refer to pacing algorithms used to pace the atria and/or ventricles in a manner that enforces a certain minimum heart rate. Because of the risk of inducing an arrhythmia with asynchronous pacing, most pacemakers for treating bradycardia are programmed to operate synchronously in a so-called demand mode where sensed cardiac events occurring within a defined interval either trigger or inhibit a pacing pulse. Inhibited demand pacing modes utilize escape intervals to control pacing in accordance with sensed intrinsic activity. In an inhibited demand mode, a pacing pulse is delivered to a heart chamber during a cardiac cycle only after expiration of a defined escape interval during which no intrinsic beat by the chamber is detected. For example, a ventricular escape interval for pacing the ventricles can be defined between ventricular events, referred to as the cardiac cycle (CC) interval with its inverse being the lower rate limit or LRL. The CC interval is restarted with each ventricular sense or pace. In atrial tracking and AV sequential pacing modes, another ventricular escape interval is defined between atrial and ventricular events, referred to as the atrio-ventricular pacing delay interval or AVD, where a ventricular pacing pulse is delivered upon expiration of the atrio-ventricular pacing delay interval if no ventricular sense occurs before. In an atrial tracking mode, the atrio-ventricular pacing delay interval is triggered by an atrial sense and stopped by a ventricular sense or pace. An atrial escape interval can also be defined for pacing the atria either alone or in addition to pacing the ventricles. In an AV sequential pacing mode, the atrio-ventricular delay interval is triggered by an atrial pace and stopped by a ventricular sense or pace. Atrial tracking and AV sequential pacing are commonly combined so that an AVD starts with either an atrial pace or sense. When used in CRT, the AVD may be the same or different in the cases of atrial tracking and AV sequential pacing.

As described above, cardiac resynchronization therapy is pacing stimulation applied to one or more heart chambers in a manner that compensates for conduction delays. Ventricular resynchronization pacing is useful in treating heart failure in patients with interventricular or intraventricular conduction defects because, although not directly inotropic, resynchronization results in a more coordinated contraction of the ventricles with improved pumping efficiency and increased cardiac output. Ventricular resynchronization can be achieved in certain patients by pacing at a single unconventional site, such as the left ventricle instead of the right ventricle in patients with left ventricular conduction defects. Resynchronization pacing may also involve biventricular pacing with the paces to right and left ventricles delivered either simultaneously or sequentially, with the interval between the paces termed the biventricular offset (BVO) interval (also sometimes referred to as the LV offset (LVO) interval or VV delay). The offset interval may be zero in order to pace both ventricles simultaneously, or non-zero in order to pace the left and right ventricles sequentially. As the term is used herein, a negative BVO refers to pacing the left ventricle before the right, while a positive BVO refers to pacing the right ventricle first. In an example biventricular resynchronization pacing mode, right atrial paces and senses trigger an AVD interval which upon expiration results in a pace to one of the ventricles and which is stopped by a right ventricular sense. The contralateral ventricular pace is delivered at the specified BVO interval with respect to expiration of the AVD interval.

Cardiac resynchronization therapy is most commonly applied in the treatment of patients with heart failure due to left ventricular dysfunction which is either caused by or contributed to by left ventricular conduction abnormalities. In such patients, the left ventricle or parts of the left ventricle contract later than normal during systole which thereby impairs pumping efficiency. This can occur during intrinsic beats and during paced beats when only the right ventricle is paced. In order to resynchronize ventricular contractions in such patients, pacing therapy is applied such that the left ventricle or a portion of the left ventricle is pre-excited relative to when it would become depolarized during an intrinsic or right ventricle-only paced beat. Optimal pre-excitation of the left ventricle in a given patient may be obtained with biventricular pacing or with left ventricular-only pacing. Although not as common, some patients have a right ventricular conduction deficit such as right bundle branch block and require pre-excitation of the right ventricle in order achieve synchronization of their ventricular contractions.

### 3. CRT pacing configuration and mode for patients with atrial conduction deficit

In certain patients, an atrial conduction deficit exists such that left atrio-ventricular synchrony does not occur during intrinsic beats even if the intrinsic atrio-ventricular interval as measured from a right atrial sense to a right ventricular sense is normal. This is exasperated by right atrial pacing and the common location of right atrial appendage pacing. Measurement of the inter-atrial conduction times has demonstrated an increase when right atrial pacing as compared with conduction of an intrinsic atrial event. Additionally there may be an abnormal conduction delay between atria in up to 20 % of CRT patients. A primary aspect of the present disclosure involves determining if such an atrial conduction deficit exists and adjusting pacing parameters accordingly. One way of determining whether an atrial conduction deficit exists is to measure the interval between a right atrial sense or pace and a left atrial sense, referred to as the RA-LA interval, as described in co-pending application Serial No. 10/920,698, entitled "BIATRIAL PACING OPTIMIZATION FOR BIVENTRICULAR PACING". A parameter which may more directly reflect the degree of asynchrony between the left atrium and left ventricle, however, is the interval between a left atrial contraction and a left ventricular contraction, referred to as the LA-LV interval.

Measurement of the LA-LV interval may be implemented by an implantable device for delivering CRT such as illustrated in Fig. 1 which has sensing/pacing channels for both atria and both ventricles. In one embodiment, after standard placement of the RA lead, an LA lead/catheter is placed either directly in the left atrium or at the upper inter-atrial septum (e.g., Backman's bundle region). The LV lead is then placed using a standard approach (e.g., in the coronary sinus or a cardiac vein). The LA-LV interval may then be measured as the time between senses generated by the electrodes of the LA lead/catheter and the LV lead during sinus rhythm and/or during pacing of the right atrium at one or more particular rates. In an alternative embodiment, a single coronary sinus (CS) lead with two sets of electrodes may be used. In this configuration, one or more electrodes will be at the distal side of the lead to sense or pace the LV, while there are one or more electrodes in the middle of the lead such that they will be disposed near the opening of the CS to sense or pace the left atrium. The LA-LV delay interval may then be measured as the time between senses generated by the distal and middle electrodes during sinus rhythm and/or during pacing of the right atrium at one or more particular rates. In still another embodiment, the LA-LV interval is measured during the LV lead implantation procedure. In this technique, the LV lead is temporarily positioned at the midportion of the coronary sinus while the lead is being implanted in order to sense the left atrium. The RA lead is also positioned. Using either the implantable device or an external device equipped with multiple sensing channels, the time interval between an RA sense or pace and an LA sense from the temporarily positioned LV is measured as the RA-LA interval. The LV lead is then further advanced through the CS to its final desired position for sensing the LV. The RA-LV delay is next measured as the interval between an RA sense and an LV sense. The LA-LV delay interval may then be computed as:

$$\text{LA-LV delay} \;=\; \text{RA-LV delay} - \text{RA-LA delay}$$

The implantable device is configured to deliver biventricular pacing in a manner specified by AVD and BVO intervals. An additional pacing parameter is also provided for pacing the left atrium, if necessary, referred to as the AAL interval, which is an escape interval triggered by a right atrial event and results in a left atrial pace upon expiration. If the measured LA-LV interval is less than a specified threshold amount, it can be surmised that a conduction deficit exists between the right and left atria, causing asynchrony between the left atrium and left ventricle. The device can therefore be configured to pace the left atrium at an AAL interval (where a zero AAL interval paces both atria simultaneously) which synchronizes left atrial and left ventricular contractions. In an alternate embodiment, the timing for left atrial pacing may be based upon left ventricular events. This involves pacing the left atrium at a specified offset interval VAL with respect to the time at which a left ventricular pace is delivered A negative VAL interval thus delivers a pace to the left atrium before the left ventricle is paced and may be used in non-atrial triggered and non-AV sequential modes as well atrial triggered and AV sequential pacing modes. In another embodiment, left atrial pacing can based upon both right atrial and left ventricular events so that both AAL and VAL intervals are defined.

4. Optimal adjustment of pre-excitation timing parameters

Once a particular resynchronization pacing configuration and mode is selected for a patient, pacing parameters affecting the manner and extent to which pre-excitation is applied must be specified. For optimum hemodynamic performance, it is desirable to deliver ventricular pacing, whether for resynchronization pacing or conventional bradycardia pacing, in an atrial tracking and/or AV sequential pacing mode in order to maintain the function of the atria in pre-loading the ventricles (sometimes referred to atrio-ventricular synchrony). Since the objective of CRT is to improve a patient's cardiac pumping function, it is therefore normally delivered in an atrial-tracking and/or AV sequential mode and requires specification of AVD and BVO intervals which, ideally, result in the ventricles being synchronized during systole after being optimally preloaded during atrial systole. That is, both optimal interventricular synchrony and optimal atrio-ventricular synchrony are achieved. As the term is used herein for biventricular pacing, the AVD interval refers to the interval between an atrial event (i.e., a pace or sense in one of the atria, usually the right atrium) and the first ventricular pace which pre-excites one of the ventricles. The AVD interval may be the same or different depending upon whether it is initiated by an atrial sense or pace (i.e., in atrial tracking and AV sequential pacing modes, respectively), The pacing instant for the non-pre-excited ventricle is specified by the BVO interval so that it is paced at an interval AVD + BVO after the atrial event. It should be appreciated that specifying AVD and BVO intervals is the same as specifying a separate AVD interval for each ventricle, designated as AVDR for the right ventricle and AVDL for the left ventricle. In patients with intact and normally functioning AV conduction pathways to the non-pre-excited ventricle, the non-pre-excited ventricle will be paced, if at all, close to the time at which that ventricle is intrinsically activated in order to achieve optimal preloading. In patients with normal AV conduction to the non-pre-excited ventricle, the optimal AVD and BVO intervals are thus related to both the intrinsic atrio-ventricular interval and the amount of pre-excitation needed for one ventricle relative to the other (i.e., the extent of the ventricular conduction deficit).

In order to optimally specify the AVD and BVO parameters for a particular patient, clinical hemodynamic testing may be performed after implantation where the parameters are varied as cardiac function is assessed. For example, a patient may be given resynchronization stimulation while varying pre-excitation timing parameters in order to determine the values of the parameters that result in maximum cardiac performance, as determined by measuring a parameter reflective of cardiac function such as maximum left ventricular pressure change (dP/dt), arterial pulse pressure, or measurements of cardiac output. Determining optimal pacing parameters for an individual patient by clinical hemodynamic testing, however, is difficult and costly. It would be advantageous if such optimal pacing parameters could be determined from data collected by the implantable device. In one approach, a variable related to cardiac output, such as transthoracic impedance, is used to compute optimum values of resynchronization pacing parameters. In one embodiment, this allows dynamic changes in device behavior to occur in response to the patient's condition through cardiac remodeling, medication changes and physiologic changes.

An implantable cardiac resynchronization device is configured to deliver biventricular pacing in an atrial tracking or AV sequential pacing mode with a specified atrio-ventricular (AVD) interval. The AVD interval (and/or BVO interval) is then varied while measuring a variable related to cardiac output such as transthoracic impedance or intracardiac ultrasound (either external or integral to the device system). The AVD interval can then be set to a value which maximizes the variable related to cardiac output. The AVD interval may be varied, for example, using a binary search algorithm to derive the value of the AVD interval which maximizes the variable related to cardiac output. As described above, an inter-atrial conduction delay which is greater than normal may make it necessary to pace the left atrium in order to achieve optimal synchronization of the left atrium and the left ventricle. The LA-LV delay interval is therefore measured by the techniques described above, with left atrial pacing initiated if the LA-LV interval exceeds a specified threshold Th1. Additionally following the optimization of the AV delay for maximum cardiac output, the LA-LV timing may be optimized in relationship to maximum cardiac output.

The techniques for setting resynchronization pacing parameters as described herein may be implemented in a number of different ways. In one implementation, a system for setting the pacing parameters includes an external programmer. In an example embodiment, a parameter related to cardiac output is measured by an implantable cardiac resynchronization device equipped with a transthoracic impedance sensor and transmitted to the external programmer via a wireless telemetry link. Either automatically or under the direction of the external programmer, the implantable device then varies the AVD and/or BVO intervals while measuring the variable related to cardiac output and measures the LA-LV interval during an intrinsic and/or right atrial paced cycle. In an automated system, the external programmer then automatically programs the implantable device with the computed optimum pacing parameter values, while in a semi-automated system the external programmer presents the computed optimum values to a clinician in the form of a recommendation. An automated system may also be made up of the implantable device alone which collects cardiac output data while varying the AVD and BVO intervals, measures the LA-LV interval and initiates left atrial pacing if necessary, determines the optimum parameter values which maximize cardiac output, and then sets the parameters accordingly. In another embodiment, which may be referred to as a manual system, the external programmer presents the collected cardiac output data and corresponding AVD and BVO intervals, as well as the LA-LV interval to a clinician for evaluation. Unless otherwise specified, references to a system for computing or setting pacing parameters throughout this document should be taken to include any of the automated, semi-automated, or manual systems just described. Another manual system could incorporate manual measurement of CO with external cardiac ultrasound while the device or external programmer optimizes the pacing parameters in a step like fashion.

5. Exemplary algorithm for computing pacing parameters and setting pacing configuration

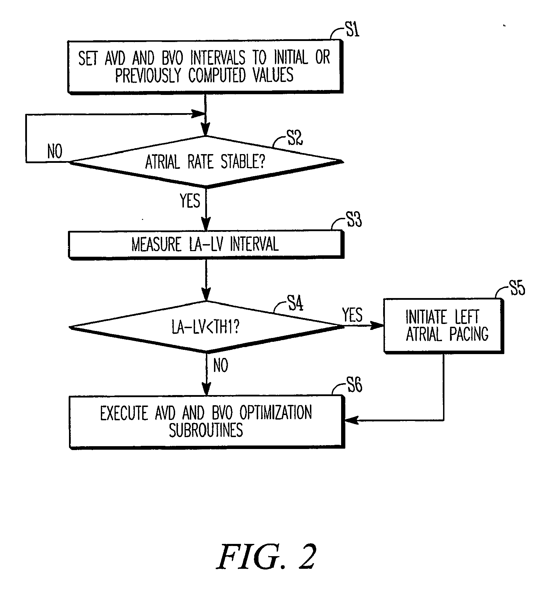

Fig. 2 illustrates an exemplary algorithm for computing the optimal pacing parameters of an implantable cardiac rhythm management device for delivering biventricular pacing. The algorithm may be partially or wholly implemented as code executed by the device controller or by an external programmer. The implantable device may be equipped with sensing and pacing channels for both ventricles and both atria as shown in Fig. 1. The device is programmed to deliver right and left ventricular paces separated by specified biventricular offset interval BVO and in an atrial tracking or AV sequential mode so that the ventricular paces are delivered at an atrio-ventricular delay interval AVD following an atrial event. At step S1, the AVD and BVO intervals are either set to nominal initial values or are as set by a previous execution of the algorithm. At step S2, the atrial rate (either paced or intrinsic) is measured and tested for stability. If the atrial rate is stable, the LA-LV interval is measured at step S3. The measured LA-LV interval may be either a single measurement or an average of LA-LV interval measurements taken over a number of beats. At step S4, the LA-LV interval is compared with a specified threshold value Th1. If the LA-LV interval is less than Th1, left atrial pacing is initiated at step S5. This will initiate left atrial contraction preceding the left ventricular event mimicking the normal physiologic events. Since this is not a 'normal' heart, minor timing variations may improve cardiac efficiency. For example in the enlarged heart and pathologically damaged left side, left atrial contraction may need to precede the left ventricular event by a greater amount than in the 'normal' healthy heart to allow full LA contraction and atrial augmentation of left ventricular filling. The algorithm then proceeds to step S6 where optimization subroutines are performed for the AVD and BVO intervals. During an optimization subroutine, the interval (either the AVD or BVO) is varied while a variable related to cardiac output is measured, with the optimum value of the interval selected as the interval value which results in maximum cardiac output.
Alternatively, if the LA-LV interval is less than Th1 and if the patient does not have intact native AV conduction allowing the use of a longer than normal AVD interval, CRT may be delivered without left atrial pacing and with a conservative AVD value which pre-excites the left ventricle later than normal. If the patient has normal AV conduction to the right ventricle, the optimum AVD value will normally be very close to the intrinsic atrio-ventricular interval and cannot be lengthened beyond it in order to compensate for an inter-atrial conduction delay. If on the other hand, the patient does not have intact native AV conduction such as complete AV block or a prolonged AV delay, it may be desirable to compensate for a prolonged inter-atrial delay with conservative AVD value rather than pacing the left atrium. In this approach, a long AVD value is used to pre-excite the left ventricle later than would be considered normal so as to maintain LA-LV synchrony even with the delayed LA contraction. A long AVD value may also be found with a cardiac output maximizing algorithm for computing an optimal AVD value. The AVD optimization subroutine, by finding the AVD value which produces maximum cardiac output, will give an AVD interval which is longer than the optimum AVD interval it would find if there were no inter-atrial delay. The optimization subroutine effectively adds the inter-atrial delay to the optimum AVD interval which would be found if there were no inter-atrial delay since it is synchrony between the left atria and ventricle that is most responsible for maximizing cardiac output.
After finding the optimum AVD as measured by cardiac output or other cardiac function, the algorithm may adjust an initial RA to LA timing (perhaps based on averaged measured patient values) through a step up/step down algorithm applied to either an AAL or VAL interval used to pace the left atrium, thus optimizing the LA-LV interval and additionally maximizing the cardiac output. The algorithm may also follow optimization of the AVD and LA-LV intervals with re-optimization of the AVD interval to confirm that it was not negatively affected by changing the inter-atrial timing.
The algorithm illustrated in Fig. 2 may be performed periodically or upon command from an external programmer. In some situations, it may be desirable for the patient to remain in a steady state during optimization of pacing parameters. Such an optimization procedure may be performed, for example, in a physician's facility or automatically by the implantable device while the patient is sleeping. Optimization of pacing parameters may also be performed during exercise (treadmill testing) since it is expected that these values will change with heart rate. This would allow the computation of dynamic optimum AV delays and inter-atrial timing as occurs in the normal physiologic heart. In this manner optimum values for the AVD, BVO, and left atrial pacing intervals may be computed for a plurality of heart rate ranges. For example, the algorithm may first determine which of a plurality of heart rate ranges corresponds to the current measured atrial rate. Optimum AVD, BVO, and left atrial pacing intervals are then computed as described above for that particular heart rate range. The device controller is then programmed to use those optimum AVD, BVO, and/or left atrial pacing interval values which have been computed for the current atrial rate.
Although the invention has been described in conjunction with the foregoing specific embodiments, many alternatives, variations, and modifications will be apparent to those of ordinary skill in the art. Other such alternatives, variations, and modifications are intended to fall within the scope of the following appended claims.

**Claims**

1. A system for setting optimal pacing parameters for delivering cardiac resynchronization therapy in a biventricular pacing mode to a patient, comprising:

    an implantable cardiac rhythm management device for delivering biventricular pacing, wherein the device is equipped with sensing and pacing channels for both atria and both ventricles and is programmed to deliver right and left ventricular paces separated by specified biventricular offset interval BVO and in an atrial tracking or AV sequential mode so that the ventricular paces are delivered at an atrio-ventricular delay interval AVD following an atrial event;
    a sensor for measuring cardiac output;
    a processor programmed to:

        1) set AVD and BVO intevals at initial values or as set by previous execution;
        2) measure and test atrial rate for stability
        3) measure the time interval between a left atrial contraction and a left ventricular contraction in the patient, designated as the LA-LV interval;
        4) compare the LA-LV interval to a specified threshold Th1 and to identify the patient as having a prolonged inter-atrial delay if the LA-LV interval is less than a threshold Th1;
        5) initiate left atrial pacing if the LA-LV interval is less than the threshold Th1;
        6) optimize the AVD and BVO intervals by varying the AVD or BVO interval while measuring a variable related to cardiac output and computing the optimum AVD or BVO intervals as the value of the AVD or BVO interval which maximizes the variable related to cardiac output;
        7) optimize the LA-LV interval by adjusting a pacing interval used to pace the left atrium wherein said pacing interval is either the atrial-atrial delay AAL following a right atriel sense or pace or a specified offset interval VAL with respect to the time at which a left ventricular pace is delivered; and,
        8) re-optimize the AVD interval to confirm that it was not negatively effected by changing inter-atrial timing.

2. The system of claim 1 wherein the processor is a controller of the implantable device.

3. The system of claim 1 wherein the processor is an external programmer configured to communicate with the implantable device via a wireless telemetry link.

4. The system of claim 1 wherein the specified AAL interval varies with heart rate.

5. The system of claim 1 wherein the specified offset interval varies with heart rate.

6. The system of claim 1 wherein the processor is further programmed to:

    compute optimum AVD, BVO, and left atrial pacing intervals for a plurality of heart rate ranges; and,
    determine which of the plurality of heart rate ranges corresponds to the current measured atrial rate and select the corresponding AVD, BVO, and left atrial pacing interval optimized for that heart rate range.


**Patentansprüche**

1. System zum Setzen optimaler Schrittgabeparameter zum Liefern einer Herzresynchronisationstherapie in einem biventrikulären Schrittgabemodus für einen Patienten, welches aufweist:

    eine implantierbare Herzrhythmus-Managementvorrichtung zum Liefern einer biventrikulären Schrittgabe, wobei die Vorrichtung mit Erfassungs- und Schrittgabekanälen für beide Atrien und beide Ventrikel versehen ist und programmiert ist zum Liefern einer rechten und einer linken ventrikulären Schrittgabe, die durch ein spezifiziertes biventrikuläres Versetzungsintervall BVO getrennt sind, und in einem atrialen Nachführungs- oder sequentiellen AV-Modus derart, dass die ventrikuläre Schrittgabe mit einem atrioventrikulären Verzögerungsintervall AVD folgend einem atrialen Ereignis geliefert wird;
einen Sensor zum Messen des Herzausgangssignals;
einen Prozessor, der programmiert ist zum:

1) Setzen der AVD- und BVO-Intervalle auf anfängliche Werte oder wie durch vorhergehende Ausführung gesetzt;

2) Messen und Prüfen der atrialen Rate für Stabilität;

3) Messen des Zeitintervalls zwischen einer linken atrialen Kontraktion und einer linken ventrikulären Kontraktion in dem Patienten, bezeichnet als das LA-LV-Intervall;

4) Vergleichen des LA-LV-Intervalls mit einem spezifizierten Schwellenwert Th1 und Identifizieren des Patienten als eine verlängerte interatriale Verzögerung aufweisend, wenn das LA-LV-Intervall weniger als ein Schwellenwert Th1 ist;

5) Initiieren einer linken atrialen Schrittgabe, wenn das LA-LV-Intervall geringer als der Schwellenwert Th1 ist;

6) Optimieren des AVD- und des BVO-Intervalls durch Variieren des AVD- oder BVO-Intervalls, während eine auf das Herzausgangssignal bezogene Variable gemessen und das optimale AVD- oder BVO-Intervall als der Wert des AVD- oder BVO-Intervalls, der die auf das Herzausgangssignal bezogene Variable maximiert, berechnet werden;

7) Optimieren des LA-LV-Intervalls durch Einstellen eines Schrittgabeintervalls, das für die Schrittgabe des linken Atriums verwendet wird, wobei das Schrittgabeintervall entweder die atriale-atriale Verzögerung AA2 folgend einer rechten atrialen Erfassung oder Schrittgabe oder ein spezifiziertes Versetzungsintervall VAL mit Bezug auf die Zeit, zu der eine linke ventrikuläre Schrittgabe geliefert wird, ist;

8) Wiederoptimieren des AVD-Intervalls, um zu bestätigen, dass es nicht negativ beeinflusst war durch Änderung des interatrialen Zeitpunkts.

2. System nach Anspruch 1, bei dem der Prozessor eine Steuervorrichtung der implantierbaren Vorrichtung ist.

3. System nach Anspruch 1, bei dem der Prozessor ein externer Programmgeber ist, der zum Kommunizieren mit der implantierbaren Vorrichtung über eine drahtlose Telemetrieverbindung ausgebildet ist.

4. System nach Anspruch 1, bei dem das spezifizierte AAL-Intervall mit der Herzrate variiert.

5. System nach Anspruch 1, bei dem das spezifizierte Versetzungsintervall mit der Herzrate variiert.

6. System nach Anspruch 1, bei dem der Prozessor weiterhin programmiert ist zum:

Berechnen der optimalen AVD-, BVO- und linken atrialen Schrittgabeintervalle für mehrere Herzratenbereiche; und

Bestimmen, welcher der mehreren Herzratenbereiche der gegenwärtigen gemessenen atrialen Rate entspricht, und Auswählen des entsprechenden AVD-, BVO- und linken atrialen Schrittgabeintervalls, das für diesen Herzratenbereich optimiert ist.

## Revendications

1. Système pour établir des paramètres de stimulation optimum pour délivrer à un patient une thérapie de resynchronisation cardiaque dans un mode de stimulation biventriculaire, comprenant :

un dispositif de gestion de rythme cardiaque implantable pour délivrer une stimulation biventriculaire, dans lequel le dispositif est équipé de canaux de détection et de stimulation pour les deux oreillettes et les deux ventricules et est programmé pour délivrer des stimulations des ventricules gauche et droit séparées par un intervalle de Décalage Biventriculaire BVO spécifié et dans un mode suivi d'oreillette ou un mode séquentiel AV de telle sorte que les stimulations ventriculaires soient délivrées selon un intervalle de Retard Atrioventriculaire AVD à la suite d'un événement d'oreillette ;
un capteur pour mesurer une sortie cardiaque ;
un processeur programmé pour :

1) établir des intervalles AVD et BVO à des valeurs initiales ou des valeurs établies par une exécution précédente ;

2) mesurer et tester la stabilité d'une fréquence d'oreillette ;

3) mesurer l'intervalle temporel entre une contraction d'oreillette gauche et une contraction de ventricule gauche chez le patient, cet intervalle étant appelé LA-LV ;

4) comparer l'intervalle LA-LV à un seuil spécifié Th1 et identifier le patient comme présentant un retard inter-oreillette prolongé si l'intervalle LA-LV est inférieur au seuil Th1 ;

5) initier une stimulation d'oreillette gauche si l'intervalle LA-LV est inférieur au seuil Th1 ;

6) optimiser les intervalles AVD et BVO en faisant varier l'intervalle AVD ou BVO tout en mesurant une variable rapportée à la sortie cardiaque et en calculant les intervalles AVD ou BVO optimum en tant que valeur de l'intervalle AVD ou BVO qui maximise la variable rapportée à la sortie cardiaque ;

7) optimiser l'intervalle LA-LV en réglant un intervalle de stimulation utilisé pour stimuler l'oreillette gauche, dans lequel ledit intervalle de stimulation est soit le Retard Oreillette-Oreillette AAL qui suit une détection ou stimulation d'oreillette droite, soit un intervalle de décalage spécifié VAL par rapport à l'instant auquel une stimulation de ventricule gauche est délivrée ; et

8) re-optimiser l'intervalle AVD afin de confirmer qu'il n'a pas été affecté négativement par la modification du cadencement inter-oreillette.

2. Système selon la revendication 1, dans lequel le processeur est un contrôleur du dispositif implantable.

3. Système selon la revendication 1, dans lequel le processeur est un programmateur externe configuré pour communiquer avec le dispositif implantable via une liaison de télémétrie sans fil.

4. Système selon la revendication 1, dans lequel l'intervalle AAL spécifié varie en fonction du rythme cardiaque.

5. Système selon la revendication 1, dans lequel l'intervalle de décalage spécifié varie en fonction du rythme cardiaque.

6. Système selon la revendication 1, dans lequel le processeur est en outre programmé pour :

calculer des intervalles AVD, BVO et de stimulation d'oreillette gauche optimum pour une pluralité de plages de rythmes cardiaques ; et

déterminer celle de la pluralité de plages de rythmes cardiaques qui correspond à la fréquence d'oreillette mesurée courante et sélectionner l'intervalle AVD, BVO et de stimulation d'oreillette gauche correspondant optimisé pour cette plage de rythmes cardiaques.

*FIG. 1*

```
                                                    ┌─S1
┌──────────────────────────────────────────┐
│  SET AVD AND BVO INTERVALS TO INITIAL OR   │
│         PREVIOUSLY COMPUTED VALUES         │
└──────────────────────────────────────────┘
                      │
        ┌─────────────┘
        │             ▼              ┌─S2
        │      ◇────────────────◇
  NO ───┤       ATRIAL RATE STABLE?
        └──────◇────────────────◇
                      │ YES
                      ▼                ┌─S3
        ┌──────────────────────────────┐
        │     MEASURE LA-LV INTERVAL     │
        └──────────────────────────────┘
                      │
                      ▼              ┌─S4          YES   ┌─S5
               ◇────────────◇ ──────────────► ┌──────────────┐
                 LA-LV<TH1?                    │ INITIATE LEFT │
               ◇────────────◇                  │ ATRIAL PACING │
                      │ NO                      └──────────────┘
                      ▼                ┌─S6              │
        ┌──────────────────────────────┐                │
        │  EXECUTE AVD AND BVO OPTIMIZATION│◄─────────────┘
        │          SUBROUTINES           │
        └──────────────────────────────┘
```

*FIG. 2*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003083700 A1
- US 2004193223 A1
- US 5902324 A

- US 5190035 A
- US 6161042 A
- US 10920698 B